# EUROPEAN PATENT APPLICATION

(11) **EP 4 223 230 A1**
(43) Date of publication of application: **09.08.2023**
(21) Application number: 21875724.3
(22) Date of filing: 29.09.2021
(51) Int. Cl.: A61B 10/00, G16H 50/20, A61B 5/11, A61B 5/113

(54) **NEUROMUSCULAR DISEASE EVALUATION SYSTEM**

(30) Priority: 29.09.2020 JP 2020163596
(71) Applicant: Mitsubishi Tanabe Pharma Corporation, Osaka 541-8505 (JP)
(72) Inventor: SAKATA, Takeshi, Osaka-shi Osaka 541-8505 (JP); GOTO, Akiko, Osaka-shi Osaka 541-8505 (JP); KATO, Asushi, Osaka-shi Osaka 541-8505 (JP); HIRAI, Manabu, Osaka-shi Osaka 541-8505 (JP)
(74) Representative: Herrero & Asociados, S.L.
(86) International application number: PCT/JP2021/035951
(87) International publication number: WO 2022/071427

(57) **Abstract**

[Problem] To provide a neuromuscular disease evaluation device that is less invasive, highly sensitive, quantitative and objective, and capable of easily evaluating neuromuscular diseases. [Solution] A device for evaluating motor functions related to neuromuscular diseases, comprising: a memory unit storing a reference value for evaluating a predetermined movement, which is calculated based on a position of at least one body part; an analysis unit for identifying a position of a body part of the user by analyzing information based on an image captured of the predetermined movement, including the user's body; and an evaluation unit for evaluating the motor function based on the numerical value calculated based on the position of the body part of the user and the reference value.

## Description

### [TECHNICAL FIELDS]

This invention relates to a neuromuscular disease evaluation system.

### [BACKGROUND ART]

Neuromuscular disease is a general term for diseases that cause movement disorders due to lesions in the brain, spinal cord, peripheral nerves, and other nerves themselves or in the muscles themselves. Typical examples include Parkinson's disease, spinocerebellar degeneration, amyotrophic lateral sclerosis, neuritis and myelitis caused by viruses and bacteria, myasthenia gravis, muscular dystrophy, and polymyositis (Non-Patent Document 1-3). Movement disorders are common primary symptoms of these neuromuscular diseases. Other diseases such as cerebral infarction, cervical spondylosis, and myelopathy, although not included in neuromuscular diseases, are symptoms that cause movement disorders and are described below as part of neuromuscular diseases.

Among neuromuscular diseases, amyotrophic lateral sclerosis (ALS), for example, is a rapidly progressive, fatal and devastating disease in which selective degenerative loss of upper and lower motor neurons results in impaired voluntary movement with progressive weakness of the upper and lower limbs, ball paralysis, and respiratory paralysis, often requiring respiratory management due to respiratory failure within 2 to 5 years of onset.

ALS is a rapidly progressive and lethal disease, and there are individual differences in the types of muscles that atrophy and their progression. Also, since ALS has no specific marker, the current diagnosis is basically exclusionary. Diagnostic criteria include, for example, the revised EL Escorial diagnostic criteria, but the diagnostic sensitivity is low and the diagnosis is actually made clinically and comprehensively. There is also electrophysiological testing, but it is difficult to diagnose ALS at an early stage with high sensitivity because it requires a large patient burden and does not track the progression or severity of the disease, so the progression of ALS is evaluated visually. Therefore, in order to evaluate limb capacity quantitatively and objectively, there is, for example, technology to improve the convenience of measuring limb capacity using motion capture technology (e.g., Patent Literature 1, etc.).

### [PRIOR ART LITERATURES]

### [PATENT LITERATURE]

[Patent Literature 1] JP6465419B2
[Non-Patent Literature 1] Japan Intractable Diseases Information Center Internet <URL: https:/ Iwww.nanbyou.or.jp/entry 15347#01 >
[Non-Patent Literature 2] The Japan Neurological Society Internet <URL: https://www.neurology-jp.org/public/disease/index.html #about>
[Non-Patent Literature 3] The Japanese Orthopaedic Association, List of symptoms of neuromuscular diseases Internet <URL: https://www.joa.org.jp/public/sick/body/nerve.html>

### [SUMMARY OF INVENTION]

### [PROBLEMS TO BE SOLVED BY THE INVENTION]

Because of the need for early detection of neuromuscular diseases to provide appropriate treatment and prevent disease progression, there is a need to improve sensitivity, quantitativity, and objectivity through the use of digital tools to easily assess neuromuscular diseases.

The purpose of this invention is to provide a neuromuscular disease evaluation system that can easily evaluate neuromuscular diseases.

### [MEANS TO SOLVE PROBLEMS]

The principal invention for solving the above problem is a device for evaluating motor functions related to neuromuscular diseases, comprising:
a memory unit for storing reference values for evaluating a predetermined movement, which are calculated based on a position of at least one body part;
an analyzing unit for identifying a position of a body part of a user by analyzing information based on an image captured of the predetermined movement, including the user's body; and
an evaluation unit for evaluating the motor function based on numerical values calculated based on the position of the body part of the user and the reference value.

Other problems disclosed by this application and their solutions will be clarified in the detailed embodiments of the Invention section and in the drawings.

### [ADVANTAGEOUS EFFECTS]

The present invention provides a neuromuscular disease evaluation device capable of evaluating neuromuscular diseases with low invasiveness, high sensitivity, and excellent quantitativity and objectivity.

### [BRIEF EXPLANATION OF DRAWINGS]

FIG. 1 shows an example of the overall configuration of a neuromuscular disease evaluation system according to this embodiment.
FIG. 2 shows an example of the hardware configuration of the server device 10.
FIG. 3 shows an example of the software configuration of the server device 10.
FIG. 4 shows an example of the user information storage unit 131.
FIG. 5 shows an example configuration of the image information storage unit 132.
FIG. 6 shows an example configuration of the reference information storage unit 133.
FIG. 7 shows an example configuration of the evaluation information storage unit 134.
FIG. 8 shows the flow of processing performed in the neuromuscular disease evaluation system according to this embodiment.
FIG. 9 shows the details of the analysis process in the process executed in the neuromuscular disease system.
FIG. 10 shows the details of the evaluation process in the process executed in the neuromuscular disease system.
FIG. 11 shows an example of the evaluation result of motor function (walking speed).
FIG. 12 shows an example of the evaluation result of motor function (arm swing angle).
FIG. 13 shows an example of the evaluation result of motor function (foot swing angle).
FIG. 14 shows an example of the evaluation results of motor function (phase difference).
FIG. 15 shows an example of the evaluation result of the motor function (neck nodding angle).
FIG. 16 shows an example of the results of evaluation of motor function (angle of back bending).
FIG. 17 shows an example of the evaluation result of the motor function (angle of body sway).
FIG. 18 show an example of the evaluation result of the motor function (relationship between the digital score and the existing index).
FIG. 19 shows an example of the evaluation results of motor function (relationship between the digital score and time).
FIG. 20 shows an example of the results of the evaluation of motor function (the relationship between the swing angle of the forward left right foot and backward left right foot and the ALSFRS-R score for gait).
FIG. 21 shows an example of the results of the evaluation of motor function (the relationship between the swing angle of the upper limb and the ALSFRS-R scores for writing, eating, and dressing).
FIG. 22 shows the relationship between upper limb movements and ALSFRS-R scores for writing.
FIG. 23 shows the relationship between the digital score for stair climbing and the ALSFRS-R scores for walking and stair climbing.
FIG. 24 shows an example of the results of the assessment of motor function (the relationship between the movable angle of ankle and the ALSFRS-R score for walking).

### [DETAILED EMBODIMENT OF THE INVENTION]

The following is a list and description of the embodiments of the invention. One embodiment of the invention comprises the following.

### [Item 1]

A device for evaluating motor function related to a neuromuscular disease, comprising:
a memory unit for storing a reference value for evaluating a predetermined movement, which is calculated based on a position of at least one body part;
an analyzing unit for identifying a position of a body part of the user by analyzing information based on an image taken of the predetermined movement, including the user's body; and
an evaluation unit for evaluating the motor function based on a numerical value calculated based on the position of the body part of the user and the reference value.

### [Item 2]

The neuromuscular disease evaluation device according to item 1, wherein the user's body part includes at least a part of the user's upper body.

### [Item 3]

The neuromuscular disease evaluation device according to item 1, wherein the user's body part includes at least the parts of the user's upper and lower body.

### [Item 4]

The neuromuscular disease evaluation device as in any one of items 2 or 3, wherein the upper body includes any one of the head, neck, back, and arms.

### [Item 5]

The neuromuscular disease evaluation device according to item 1, wherein the evaluation unit evaluates the motor function based on items related to the motor function in the examination of neuromuscular diseases.

### [Item 6]

The neuromuscular disease evaluation device according to item 1, wherein the evaluation unit evaluates the motor function based on items related to the motor functions in a test for amyotrophic lateral sclerosis.

### [Item7]

The neuromuscular disease evaluation device according to item 1, wherein the evaluation unit evaluates the motor function based on items related to the coordinating movement of the user's parts.

### [Item 8]

The neuromuscular disease evaluation device according to item 1, wherein the evaluation unit evaluates the motor function based on items related to the motor function included in the ALS Functional Rating Scale (ALSFRS-R).

### [Item 9]

The neuromuscular disease evaluation device according to item 1, wherein the evaluation unit includes evaluating for muscle atrophy.

### [Item 10]

The neuromuscular disease evaluation device according to item 5, wherein the item includes at least one of the following selected from language, salivation, swallowing, writing, eating actions, dressing activity/body movements, movements in bed, walking, climbing stairs, dyspnea, orthopnea, and respiratory failure.

### [Item 11]

The neuromuscular disease evaluation device according to item 1,
wherein the analyzing unit analyzes an image of the user's body in the user's walking movement as the predetermined movement; and
the evaluation unit evaluates the walking movement and/or the quality of the walking movement as an evaluation of the motor function.

### [Item 12]

The neuromuscular disease evaluation device according to item 11, wherein in connection with the evaluation of the quality of the walking movement, the user is evaluated for at least one of the following: walking speed, stride length, arm swing, leg swing, phase difference of each joint marker of the whole body, neck nod, back bend, body side swing, body wobble (front to back and side to side).

### [Item 13]

The neuromuscular disease evaluation device according to item 11, wherein at least one of the following is evaluated in connection with the evaluation of the quality of the walking movement: walking speed, arm swing, leg swing, phase difference of each joint marker of the whole body, neck nodding, back bending, and body side swing.

### [Item 14]

The neuromuscular disease evaluation device according to item 11, wherein the evaluation is performed in connection with the walking movements and with items related to motor function included in the ALS Functional Rating Scale (ALSFRS-R).

### [Item 15]

The neuromuscular disease evaluation device according to item 1, wherein the position relates to a joint.

### [Item 16]

The neuromuscular disease evaluation device according to item 1,
wherein the analyzing unit analyzes an image of the user's body in the user's walking movement as the predetermined movement; and
the evaluation unit evaluates the writing, the eating, or the dressing/body movements and/or quality as the evaluation of the motor function.

### [Item 17]

The neuromuscular disease evaluation device according to item 16, wherein the user's arm swing is evaluated in conjunction with the evaluation of the quality of writing, eating actions, or dressing activity/body movement activities.

### [Item 18]

The neuromuscular disease evaluation device according to item 1, wherein the evaluation unit includes determining the degree of progression of the neuromuscular disease based on the evaluation.

### [Item 19]

The neuromuscular disease evaluation device according to item 1, wherein the evaluation unit includes determining a patient group based on the progression of motor dysfunction based on the evaluation.

### [Item 20]

The neuromuscular disease evaluation device according to item 1, wherein the neuromuscular disease includes amyotrophic lateral sclerosis.

### [Item 21]

The neuromuscular disease evaluation device according to item 1, wherein the evaluation unit evaluates the range of motion of the joints.

### [Item 22]

The neuromuscular disease evaluation device according to item 1, wherein the analysis unit includes identifying the body part of the user by motion capture technology.

### [Item 23]

The neuromuscular disease evaluation device according to item 1, wherein the neuromuscular diseases include one of Parkinson's disease, Huntington's disease, amyotrophic lateral sclerosis, spinocerebellar degeneration, progressive supranuclear palsy, multiple system atrophy, multiple sclerosis, neuromyelitis optica, Adrenoleukodystrophy, metachromatic leukodystrophy, hyper glutaric acidemia type I, phenylketonuria, GM1 gangliosidosis, GM2 gangliosidosis, mucolipidosis type II (I-cell disease), Angelman syndrome, Krabbe disease, Batten disease, mucopolysaccharidosis, Rett disease, Niemann-Pick disease A,B,C, spinal cord injury, inclusion body myositis, myasthenia gravis, hereditary spastic paraplegia, primary lateral sclerosis, Charcot-Marie-Tooth disease, spinal muscular atrophy, Friedreich's ataxia, dermatomyositis, polymyositis, Guillain-Barre syndrome, chronic inflammatory, demyelinating polyneuritis, Lambert-Eaton myasthenia, multifocal motor neuropathy, anti-MAG peripheral neuropathy, facioscapulohumeral dystrophy, muscular dystrophy, myotonic dystrophy, Duchenne muscular dystrophy, facioscapulohumeral muscular dystrophy, spinal and bulbar atrophy, mitochondrial disease, Leigh's encephalomyelopathy, MELAS (Mitochondrial myopathy, Encephalopathy, Lactic acidosis, stroke-like episodes syndrome), fragile X-associated tremor/ataxia syndrome (FXTAS), Pelizaeus-Merzbacher disease (PMD), neuritis or myelitis caused by a virus or bacteria, cerebral infarction, cervical spondylosis, or spondylosis.

### [Item 24]

The neuromuscular disease evaluation device according to item 16, wherein at least one of the following is evaluated in connection with the evaluation of the quality of the gait movement: the user's forward left-right foot and backward left-right foot swing, the forward left-right arm and backward right-right arm swing, and the angle of ankle motion.

The following is a description of the embodiment of the present disclosure with reference to the drawings. In this specification and the drawings, duplicate explanations are omitted for components that have substantially the same functional configuration by applying the same symbols.

### <System Configuration>

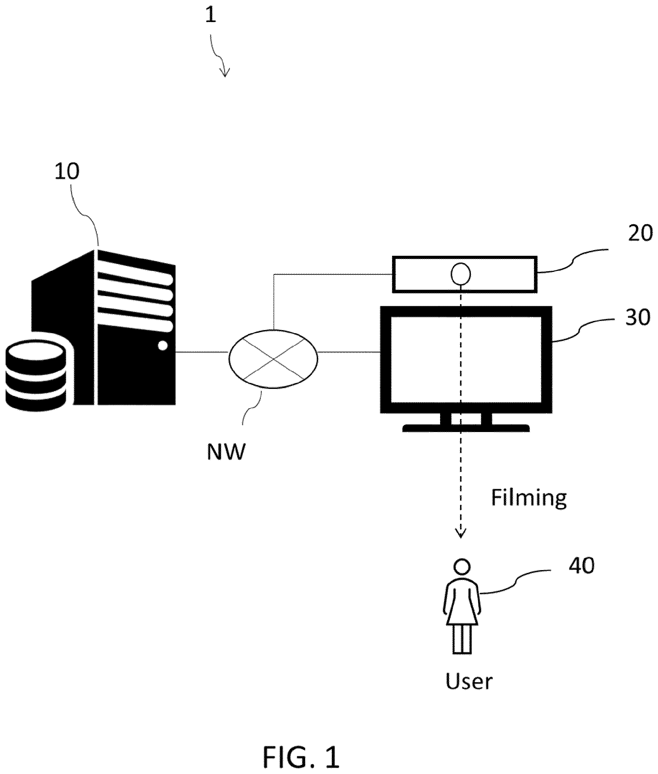

FIG. 1 shows an example configuration of a neuromuscular disease evaluation system (hereinafter also referred to as "the System") in accordance with an embodiment of the present disclosure. A camera captures images of a user, such as a patient, performing physical exercise, and evaluates the user's neuromuscular disease-related motor function from the captured images (which may be still images or moving images, but in this embodiment, the images are assumed to be moving images).

FIG. 1 shows an example of the overall configuration of a neuromuscular disease evaluation system of this embodiment. As shown in FIG. 1, the neuromuscular disease evaluation system 1 of this embodiment has a server device 10, a camera 20, and a display 30 connected to each other via a communication network NW. The communication network NW is, for example, the Internet or LAN (Local Area Network), and is constructed by a public telephone line network, a dedicated telephone line network, a cellular telephone line network, Ethernet (registered trademark), wireless communication channel, etc.

The server device 10 is a computer that manages the entire neuromuscular disease evaluation system and evaluates motor function. The server device 10 is, for example, a workstation, a personal computer, or a virtual computer logically realized by cloud computing. The server device 10 receives video images captured by the camera 20 and analyzes the received video images to evaluate physical exercise.

Although not shown in the figure, the server device 10 can further connect to an external server device via a network, transmit the information acquired by the camera 20 through image capture and analyzed information to the external server device, and receive the information analyzed by the external server device.

The camera 20 captures images of a user 40 who is undergoing neuromuscular disease evaluation and detects information indicating each part of the patient (e.g., information indicating virtual markers corresponding to each part) based on the moving images captured of the user 40. The camera 20 can use motion capture, for example, a Kinect (registered trademark) sensor from Microsoft Corporation. The camera 20 can transmit information based on the captured moving images to the server device 10 via protocols such as TCP/IP and Bluetooth (registered trademark).

The display 30 has the similar configuration as known displays, such as a liquid crystal display, and displays images based on various image data obtained from the camera 20. The display 30 outputs information under control from the server device 10 via the communication network NW. By connecting a computer to the display 30 and having the computer communicate with the server device 10, information can be output to the display 30 in response to instructions from the server device 10. The display 30 can show the user 40 a guideline for what action to take when capturing a moving image of the user 40 with the camera 20. For example, a background video image including the user 40 can be displayed on the display 30, and the user 40 can be shown at a predetermined distance (e.g., 10 meters or 4.5 meters) and/or for a predetermined time period performing predetermined actions (e.g., speech, salivation, swallowing, writing, eating actions, dressing/body movements, sleeping movements, walking, climbing stairs, dyspnea, orthopnea, and respiratory failure, etc.) can be guided using text, reference lines, icons, or other graphical displays.

Although not shown in the figure, the system can include user a terminal such as smartphones, personal computers, game consoles, etc., managed by the user 40. The user terminal can have an application program installed, and the computing unit of the user terminal can execute all or some of the functions performed by the server device 10. The information stored in a storage unit 103 provided in the server device 10 can be made to be stored in the memory unit built into the user terminal. The system can also include an evaluator terminal, such as a smartphone, personal computer, or the like, managed by an evaluator, such as a physician, who conducts the evaluation of the exercise function of the user 40. Similar to the user terminal, the evaluator terminal can make the evaluator terminal to perform all or some of the functions performed by the server device 10, and can make the evaluator terminal store the information stored in the storage unit 103 provided in the server device 10.

Next, the details of the configuration of the above-mentioned neuromuscular disease evaluation system 1 will be described.

### <Hardware Configuration>

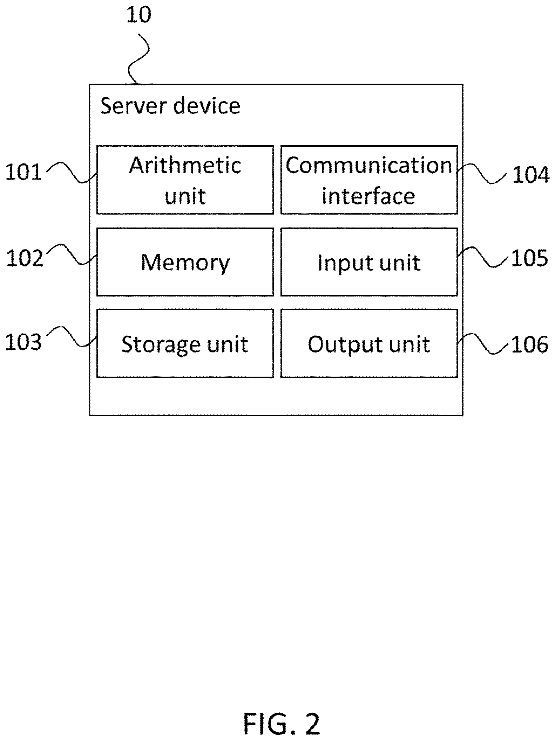

FIG. 2 shows an example of the hardware configuration of the computer that realizes the server device 10 in this embodiment.

The server device 10 is comprised of an arithmetic unit 101, a memory 102, a storage unit 103, a communication interface 104, an input unit 105, and an output unit 106. These are electrically connected to each other through a bus (not shown). As described above, all or part of the functions performed by the storage unit 103 and other units and the arithmetic unit 101 included in the server device 10 can be provided or performed by other devices, such as user terminals.

The arithmetic unit 101 controls the operation of the server device 10 as a whole, controls the transmission and reception of data between each element, and performs information processing necessary for application execution and authentication processing. The arithmetic unit 101 is a processor, such as a CPU (Central Processing Unit) or GPU (Graphics Processing Unit), for example, and executes programs, etc. stored in the storage unit 103 and deployed in the memory 102 to perform each information processing.

The memory 102 includes a main memory comprising a volatile storage device such as DRAM (Dynamic Random Access Memory) and an auxiliary memory comprising a non-volatile storage device such as flash memory or HDD (Hard Disc Drive). The memory 102 is used as a work area or the like for the arithmetic unit 101. It also stores the BIOS (Basic Input/Output System) and various configuration information, etc. that are executed when the server device 10 is started.

The storage unit 103 stores various programs such as various data and application programs. For example, it may be a hard disk drive, solid state drive, flash memory, etc.

The communication interface 104 is an interface for connecting the server device 10 to the communication network NW, such as an adapter for connecting to an Ethernet (registered trademark), a modem for connecting to a public telephone network, a wireless communicator for wireless communication, a USB (Universal Serial Bus) connectors and RS132C connectors for serial communication, etc., to connect to a network.

The input unit 105 is a keyboard, a mouse, a touch panel, a button, a microphone, etc., that inputs data.

The output unit 106 is a display, a printer, a speaker, etc. that outputs data, for example.

### <Software configuration>

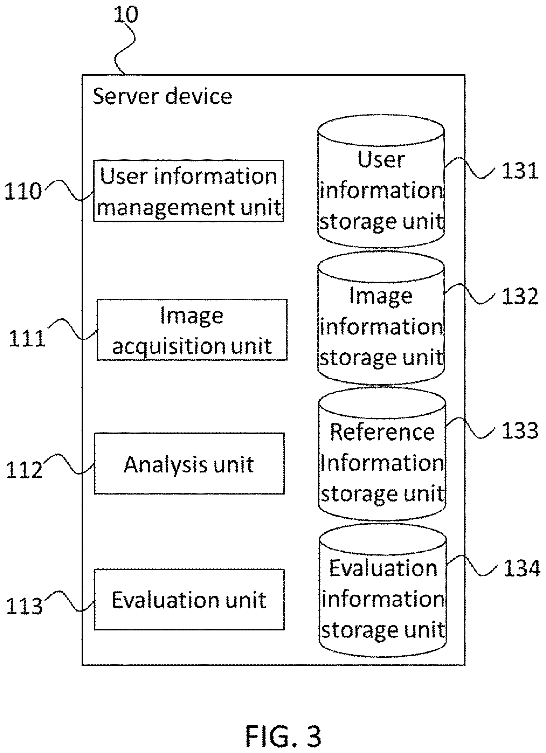

FIG. 3 shows an example of the software configuration of the server device 10 in this embodiment. As shown in FIG. 3, the server device 10 has each of the following functional units: user information management unit 110, image acquisition unit 111, analysis unit 112, and evaluation unit 113, and each of the following storage units: user information storage unit 131, image information storage unit 132, reference information storage unit 133, and evaluation information storage unit 134.

Each of the above functional parts is realized by the arithmetic unit 101 provided by the server device 10 reading a program stored in the storage unit 103 into the memory 102 and executing it, and each of the above memory parts is realized as part of the memory area provided by the memory 102 and storage unit 103 provided by the server device 10.

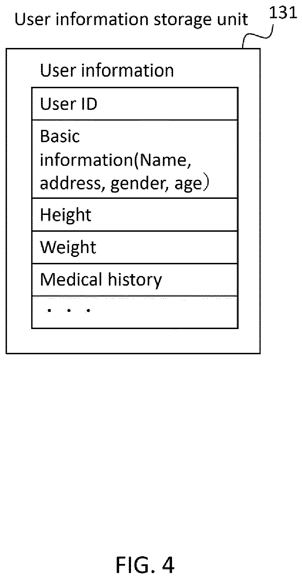

The user information storage unit 131 stores information about the user 40. FIG. 4 shows an example of the structure of the user information storage unit 131. As shown in FIG. 4, the user information stored by the user information storage unit 131 includes basic information (personal information such as name, address, gender, age, etc.), physical information such as height, weight, etc., information such as medical history, progression, etc. related to neuromuscular and other diseases such as amyotrophic lateral sclerosis (ALS), spinocerebellar degeneration, Huntington's disease and Parkinson's disease, etc.

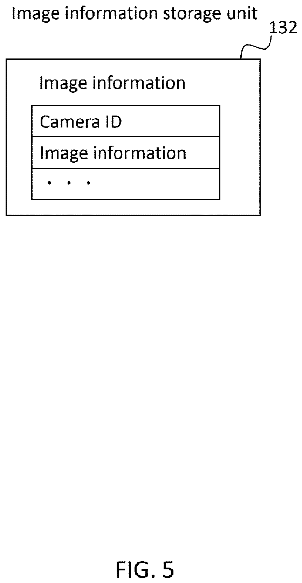

The image information storage unit 132 stores image information such as moving images captured by the camera 20. FIG. 5 shows an example configuration of the image information storage unit 132. As shown in FIG. 5, the image information storage unit 132 stores image information, and the image information is associated with a camera ID that identifies the camera 20 that captured the image. The image information is data for displaying moving images captured by the camera 20.

The image data can be in any format, such as MP4, MPEG2, AVI, MOV, etc. The image information is not limited to the image itself, but can also include information based on the image, such as the position coordinates of the user's body parts, or the like, obtained from the image. Furthermore, the image information can also include information about virtual markers extracted from the image.

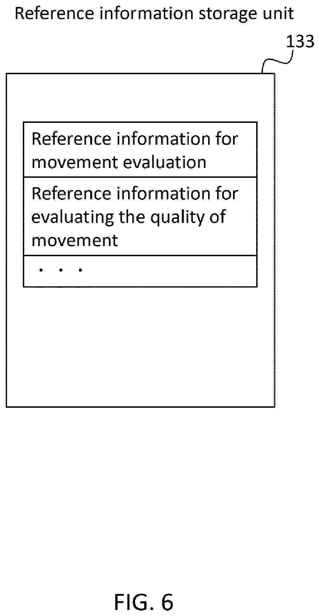

The reference information storage unit133 stores reference information (information for evaluating movements calculated based on reference values, including calculation formulas, hereinafter referred to as "reference information") that is referred to when evaluating the motor function of the user 40. FIG. 6 shows an example configuration of the reference information storage unit 133. As shown in FIG. 6, the reference information storage unit 133 stores, for example, the reference information for movement evaluation (e.g., when the user's motor function is evaluated to measure the onset and/or progression of ALS, information on the evaluation of movement that is listed as an item to be evaluated in the ALS Functional Rating Scale (hereinafter referred to as the "ALSFRS -R") and the reference information for evaluating the quality of movement (e.g., if the movement is walking, information on the evaluation of walking speed, stride length, arm swing, back bend, body swing, etc.) to be referenced to evaluate the quality of a predetermined movement of the user.

Here, ALSFRS-R is a comprehensive severity index for ALS patients. ALSFRS-R looks at activities of daily living of ALS patients and consists of four parts: ball function, upper limb ADLs, lower limb ADLs, and respiratory status. Each of 12 activities is scored on a 5-point scale from 0 to 4 to evaluate the overall severity and progression of the disease in ALS patients.

The 12 items evaluated are: 1) speech, 2) salivation, 3) swallowing, 4) writing, 5) eating, 6) dressing/body movement, 7) body movement in bed, 8) walking, 9) climbing stairs, 10) dyspnea, 11) orthopnea, and 12) respiratory failure. In addition to ALSFRS-R, other ALS assessment methods include the 40-item Amyotrophic Lateral Sclerosis (ALS) Assessment Questionaire (ALSAQ-40), Japanese ALS Severity Classification, Modified Norris Scale, etc. The clinical movement values to be quantitatively evaluated in the present invention can be utilized for some or all of the evaluation items for neuromuscular diseases other than ALS, such as spinocerebellar degeneration. For example, when evaluating a user's motor function as a disease other than ALS to measure the onset and/or progression of spinocerebellar degeneration, the Scale for the assessment and rating of ataxia (SARA) includes 1) walking, 2) standing, 3) sitting, 4) speech impairment, 5) finger pursuit, 6) finger-nose, 7) hand pronation and supination, and 8) heel to shin as assessment items. When performed to measure the onset and/or progression of Huntington's disease, the Unified Huntington's Disease Rating Scale (UHDRS) includes 1) Motor Assessment, 2) Cognitive Assessment, 3) Behavioral Assessment, 4) Independence Scale, 5) Functional Assessment, and 6) Total Functional Capacity (TFC). In addition, when performed to measure the onset and/or progression of Parkinson's disease, the Unified Parkinson's Disease Rating Scale (UPDRS) may include all or part of the following items: 1) mental function, 2) activities of daily living, 3) motor function, and 4) side effects. It can also be used to measure the onset and/or progression of diseases exemplified as neuromuscular diseases, as described below. Existing indices used to measure the onset, etc. of the diseases exemplified above are collectively referred to as "existing indices.

In addition, each of the above indices can be utilized for some or all of the movements included in the Functional Independence Measure (FIM), which is one of the evaluation indices of ADLs. For example, the movements to be evaluated included in the FIM include arm rolling, standing up, standing on one leg, squatting, and so on. Hereafter, the FIM is also referred to as the Existing Indices.

The sreference information for movement evaluation includes, for example, statistical evaluation information. Statistical evaluation information refers to numerical values calculated using an optimal formula that can show statistically significant differences. Here, in generating statistical evaluation information, information regarding the virtual markers corresponding to each part of the user's body captured by the camera 20 (For example, based on changes in the position coordinates over time of virtual markers corresponding to up to 25 different sites in relation to the user's video image, which are acquired every 1/30 second via KINECT (registered trademark), the virtual markers necessary to determine the significant difference in movement and the characteristics of movement for each movement such as walking (for example, if the movement is a walk) (e.g., if the movement is walking, the site for determining the significant difference in gait (normal gait vs. slow gait)) is selected, a calculation formula that can capture significant differences and characteristics of each movement using the virtual markers is created, and the numerical values calculated using the calculation formula are compared with the numerical values evaluated based on the existing clinical indices used by doctors and others for evaluation. The formula is also used to generate the optimal formula for evaluating the onset and/or progression of neuromuscular disease, etc. by having the patient's movement of neuromuscular disease, etc. quantified and comparing the numerical values with the numerical values evaluated based on existing clinical indices that physicians, etc. use for evaluation. This calculation formula, together with the reference values, can be stored in the server device 10 as reference information for movement evaluation. Here, the reference value refers to the value of the user's movement, the measured value itself, which is used as a reference in evaluating significant differences in movement. Instead of the reference information for movement evaluation, a doctor or other person may evaluate the significant difference of the user's movement based on the above existing indices by directly observing the user's movement or by visually observing the user's movement captured by the camera 20.

Here, as in the above, in generating the statistical evaluation information, information on virtual markers corresponding to each part of the user's body captured by the camera 20 (e.g., virtual markers corresponding to up to 25 different parts of the body in relation to the user's moving image, which are acquired every 1/30 second via KINECT (registered trademark)) is used to generate the statistical evaluation information. Based on the changes in the position coordinates of the markers over time, the virtual markers necessary to determine the characteristics of each motion such as walking (e.g., if the motion is walking, the part of the body used to determine the characteristics of the motion such as upper body leaning, small stride length, etc. based on walking speed and stride length) are selected for each motion such as walking, and a formula is created to capture the characteristics of each motion as the quality of the motion using the virtual markers. Then, the optimal formula is generated, which can show statistically significant differences by comparing the values calculated using the formula with the values evaluated based on existing clinical indices used by physicians and others for evaluation. The formula is also used to generate the optimal formula for evaluating the onset and/or progression of neuromuscular disease, etc., by having the patient's movement of neuromuscular disease, etc., quantified and comparing the numerical values with the numerical values evaluated based on existing clinical indices that physicians, etc., use for evaluation. This calculation formula, together with the reference value, can be stored in the server device 10 as reference information for evaluating the quality of movement. Here, the reference value refers to the value of the user's movement, the measured value itself, which is used as a standard in evaluating significant differences in movement. For example, for walking speed, the head, neck, spine shoulder, spine mid, and spine base are selected as virtual markers to be used when calculating walking speed, and the numerical values indicating walking speed calculated based on changes in the position coordinates of these virtual markers over time in the frontal direction (Z-axis) with the camera 20 as the origin based on a predetermined formula are compared with the numerical values evaluated based on existing clinical indices used by physicians and others for evaluation. As a result, an optimal calculation formula can be generated that can show statistically significant differences, and the walking speed can be calculated from the optimized formula. The formula is also used to generate the optimal formula for evaluating the onset and/or progression of neuromuscular disease by having the quality of movement of patients with neuromuscular disease quantified and compared to values evaluated based on existing clinical indices used by physicians and others for evaluation. Similarly, for stride length, hip and knee are selected as candidates for virtual markers, an approximate formula for the centerline is generated using the Z-axis as the centerline as the relative coordinate centered on the hip, then the stride length is calculated based on the distance between the centerline and each displacement based on the displacement of the position coordinate of the knee over time around the centerline. The values can be compared with the values evaluated based on existing clinical indices used by physicians and others for evaluation, and an optimal formula can be generated that can show statistically significant differences, and the stride length can be calculated from the optimized formula. The formula can be stored in the server device 10 as reference information for evaluating the quality of movement, along with the numerical values that serve as reference values. Similarly, for arm swing, shoulder and elbow were selected as candidates for virtual markers, and an approximate formula is generated using the size of the angle between shoulder and elbow as an index and the Z-axis as the center line as a relative coordinate centered on the shoulder. The arm swing is calculated based on the distance between the centerline and each displacement based on the displacement of the positional coordinates of the elbow around the centerline over time, the values are compared with the values evaluated based on existing clinical indices used by doctors and others for evaluation, and an optimal calculation formula that can show statistically significant differences is generated. The arm swing can be calculated from the optimized formula. The calculation formula can be stored in the server device 10 as reference information for evaluating the quality of movement, together with the numerical values that serve as reference values. For back bending (forward leaning posture), the head, spine shoulder, and spine base are selected as candidate virtual markers, the angles of the head and spine shoulder to the ground and the angles of the spine shoulder and spine base to the ground are used as indices, and the back bending is calculated based on predetermined formulas. The value is compared with the value evaluated based on existing clinical indices used by physicians and others for evaluation, to generate an optimal formula that can show statistically significant differences, and from that optimized formula the back bending (forward leaning posture) can be calculated. The formula can be stored in the server device 10 as reference information for evaluating the quality of movement, together with a numerical value that serves as the reference value. For body sway, the spine base is selected as a candidate virtual marker, and an approximate equation is generated using the angle between the spine base and the ground as an index, with the X axis as a relative coordinate centered on the ground, and the displacement of the positional coordinates of the spine base over time around the center line. The optimal formula can be generated based on the distance between the centerline and each displacement based on the displacement of the position coordinates around the centerline over time, the value is compared with the value evaluated based on existing clinical indices used by doctors and others for evaluation, and the body sway can be calculated from the optimized formula that can show statistically significant differences. The calculation formula can be stored in the server device 10 as reference information for evaluating the quality of movement, together with numerical values that serve as reference values. Furthermore, for the phase difference between the upper body (hands, etc.) and lower body (feet, etc.), predetermined parts of the hands and feet can be selected as candidates for virtual markers, the phase difference between the predetermined parts of the hands and feet can be calculated based on the predetermined calculation formula, the values can be compared with the values evaluated based on existing clinical indicators used by doctors, etc. for evaluation, and statistically significant differences. Then, the optimal formula can be generated and the phase difference between the upper body (e.g., hands) and lower body (e.g., feet) can be calculated from the optimized formula. The calculation formula can be stored in the server device 10 as reference information for evaluating the quality of movement, together with numerical values that serve as reference values.

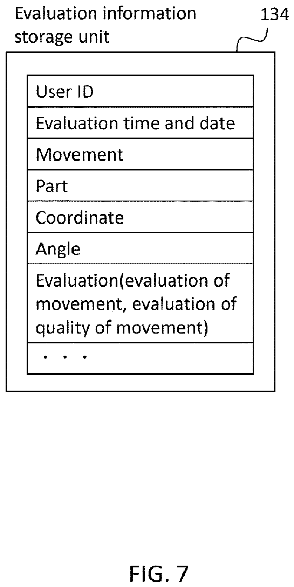

The evaluation information storage section 134 stores information indicating the evaluation of the motor function of the user 40 (hereinafter referred to as "evaluation information"). FIG. 7 shows an example of the evaluation information storage section FIG. 7 shows an example configuration of the evaluation information storage unit (134). As shown in FIG. 7, the evaluation information includes, corresponding to the user ID of each user, the date and time of the evaluation of movements, the contents of movements (e.g., movements related to items to be evaluated in the ALSFRS-R, such as walking, swallowing, eating, dressing, climbing stairs, and breathing, if the neuromuscular disease to be tested is ALS), various information used for evaluation of significant differences in movement and evaluation of the quality of movement, for example, the user's virtual markers (parts), the position coordinates of the parts that change over time, the relative angle of the part to other parts, and information on the evaluation of movement and quality of movement (e.g., numerical values calculated based on predetermined formulas or the like). Here, each evaluation information corresponding to multiple evaluation dates and times is stored in correspondence with the user ID, and by referring to the user's past evaluations, the progress of the user's neuromuscular disease can be evaluated.

In evaluating the motor function, the user information management unit 110 accepts input of information about the user by the evaluator, etc. via the input unit (not shown) of the server device 10 and stores the information in the user information storage unit 131. In evaluating the user's movement and the quality of the user's movement as the user's movement evaluation, the image acquisition unit 111 acquires a moving image of the user performing a predetermined movement that is captured by the camera 20, the analyzing unit 112 identifies the user's movements from information based on the acquired moving images, such as the position coordinates of the user's body parts, determines virtual markers corresponding to the identified movements, and calculates numerical values based on the position coordinates of the virtual markers using the calculation formulas included in the above-mentioned reference information for movement evaluation, the evaluation unit 113 determines the score for evaluation by referring to the numerical value stored as the reference value included in the reference information for evaluating the movement. When the neuromuscular disease to be tested is ALS, as the evaluation item included in the ALSFRS-R, as the movement evaluation, the evaluation is performed on the significant difference of a given movement, for example, by a 5-level score from 0 to 4 (for example, evaluated with scores of 0, 1, 2, 3, and 4). In addition, as a quality evaluation of the movement, a digital evaluation item (e.g., if the movement is walking, the speed of walking, stride, arm swing, back bend, body side swing, etc.) is evaluated in terms of its quality (e.g., if the movement is walking, based on each of the following values: walking speed, stride length, arm swing, back bend, and body side swing) and the score for the movement are determined. The onset and/or progression of the user's neuromuscular disease is determined based on the score for evaluating the user's movement and the score for evaluating the quality of movement, and stored as evaluation information. In addition, since the analyzing unit 112 evaluates the motor function based on items related to the cooperative movement of the parts of the user 40 (where "cooperative movement" refers to movement based on "nerve-muscle coordination" or "muscle-muscle coordination"), the cooperative movement can be analyzed with reference to multiple parts of the user 40.

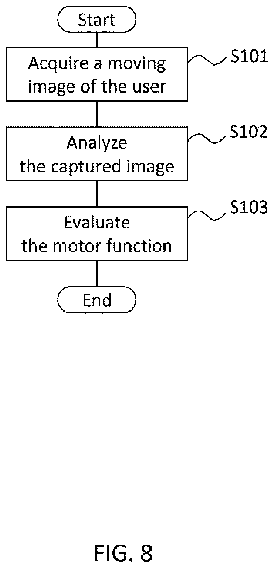

FIG. 8 shows the flow of processes performed in this neuromuscular disease evaluation system.

First, as a pre-processing of this process, the user information management unit 110 of the server device 10 accepts information input concerning the user to be the subject and stores the user information in the user information storage unit 131. Alternatively, if the user information has already been stored in the user information storage unit 131 when evaluating the user's motor ability, the user information management unit 110 can refer to the user information necessary for evaluating motor ability by accepting input such as user ID or the like.

Next, as step S101, the image acquisition unit 111 acquires a moving image of the user 40 captured by the camera 20. Here, the user 40 can be instructed in advance by the evaluator or through a guide displayed on the display 30 as to what movements should be performed in front of the camera 20 when evaluating motor function for testing for neuromuscular diseases. For example, when evaluating motor function to measure the presence and/or progression of ALS as a neuromuscular disease, the user 40 can be instructed to perform walking movements as one of the evaluation items included in the ALSFRS-R. For example, the user 40 can be instructed to walk toward the camera for a distance of 4.5 meters along the frontal direction (Z-axis) with the camera 20 as the origin, as a walking movement. Alternatively, the user 40 can be instructed to perform a 2-minute walking motion, changing the distance requirement. Thus, in the case of conventional visual inspection, a 10-meter walking motion was required of the user to capture the characteristics of the user's movements. According to this embodiment, however, since the user's movements can be analyzed digitally using virtual markers, the inspection can be performed by having the user walk a relatively short distance, such as 4.5 meters.

Here, the camera 20 is equipped with a sensor for motion capture (e.g., KINECT (registered trademark)) to capture the walking motion of the user 40. Here, non-invasive sensors can be used to detect user motion, and the user 40 does not need to wear any particular motion capture sensors. By capturing images with camera 20, up to 25 virtual markers corresponding to the user's body parts are recognized, and the values indicated by the position coordinates of the virtual markers in the frontal direction (Z axis), right direction (X axis), and upward direction (Y axis) with camera 20 as the origin can be obtained every 1/30 second for the duration of the image capture. The camera 20 is not limited to this and can be, for example, a camera connected to a game device used as a user terminal or evaluator terminal, a digital camera built into a smartphone, or the like. The image acquisition unit 111 stores the acquired moving images as image information in the image information storage unit 132. By referring to the position coordinates of the user's virtual marker in the three axes (X, Y, and Z axes) as described above, the detection accuracy can be improved for a given movement, including the inclination of the user's movement.

Next, as step S 102, the analysis unit 112 analyzes the acquired video image. The details of the analysis process are described below.

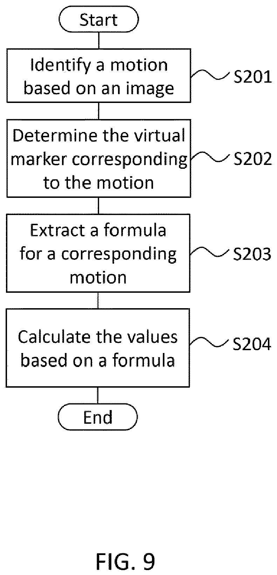

FIG. 9 shows a detailed example of the analysis process by the analysis unit 112. As shown in step S201 in FIG. 9, as the analysis process, the analysis unit 112 identifies the actions of the user 40 based on the image information. For example, the analysis unit 112 identifies the position coordinates of the virtual markers of the user 40's body and their changes over time from the image information stored in the image information storage unit 132, and identifies specific actions of the user 40 based on the feature values. As an example, the analysis unit 112 can identify that the user 40 is performing a walking motion. Here, as another example, the analysis unit 112 can also identify the movement of the user 40 based on the image-based information comprising the information of the virtual marker extracted from the image.

Next, as shown in step S202, the analysis unit 112 determines the virtual marker corresponding to the identified movement. If the identified movement is a walking movement, the analysis unit 112 determines the virtual markers necessary for evaluating the walking movement. For example, in evaluating the significance of the walking motion or the quality of the walking motion, the analysis unit 112 can determine virtual markers corresponding to at least the parts of the user's upper body (e.g., one or a combination of the head, neck, back, and arms). For example, if the motion is walking, head, neck, spine shoulder, spine mid back, and spine base can be determined as candidate virtual markers for walking speed. The virtual markers used in the analysis should be minimal, considering the burden of movement by the user and the amount of information that can be obtained from the image information, and should correspond to a part of the body, such as either the upper or lower body, rather than the entire body. In particular, when analyzing walking motions, at least the virtual markers corresponding to the parts of the body included in the upper body can be determined and analyzed. For example, in step S101, the camera 20 can capture the motion of the upper body of the user 40 seated in a chair or the like, and analyze the motion related to the walking motion, such as arm swinging, by referring only to the upper body part of the user 40. The analysis unit 112 can also determine virtual markers corresponding to the parts of both the upper and lower body for more accurate analysis.

Next, as shown in step S203, the analysis unit 112 refers from the reference information storage unit 133 the reference information for evaluating movements and the reference information for evaluating the quality of movements necessary for evaluating the quality of movements, respectively, corresponding to the movements identified above. The reference information for evaluating movement and the reference information for evaluating the quality of movement each contain a calculation formula for evaluating movement and a calculation formula for evaluating the quality of movement, respectively. For example, if the movement is walking, a formula for calculating walking speed can be extracted to evaluate the quality of the movement.

Next, as shown in step S204, the analysis unit 112 uses the virtual markers identified in step S202 to calculate numerical values that are referred to for evaluating movement and quality of movement based on the calculation formulas included in the reference information for movement evaluation and in the reference information for movement quality evaluation. For example, to calculate a numerical value for evaluating a significant difference in walking, the analysis unit 112, for example, calculates a numerical value indicating a significant difference in walking based on the formulas included in the reference information for evaluating movement, using the virtual marker included in the upper body. To calculate the walking speed, the analysis unit 112, for example, uses the position coordinates of virtual markers such as head, neck, spine shoulder, spine mid back, and spine base of the user 40 that change over time in the Z-axis direction to calculate the walking speed. The walking speed can be calculated based on a predetermined formula. For example, the analysis unit 112 can calculate the stride length based on the distance between the centerline and each displacement, using the size of the angle formed by the user 40's hip and knee as an index, the Z-axis as a relative coordinate centered on the hip, and the displacement of the position coordinates of the knee over time around the centerline. This can be calculated based on a predetermined formula using this method. Here, the feature is that the stride length is calculated using, for example, the size of the angle formed by the hip and knee as an index, in order to absorb individual differences in height and leg length among patient users, as described above. For example, the stride length may be calculated based on the relative positions of the hip and ankle. However, since the position of the ankle is relatively displaced by the position of the knee, it is preferable to calculate the stride based on the relative positions of the hip and knee, which are in close proximity. Similarly, for arm swing, the analysis unit 112, for example, uses the size of the angle formed by the shoulder (shoulder) and elbow (elbow) of the user 40 as an index, and the Z axis as a relative coordinate centered on the shoulder (shoulder) as the center line. Based on the displacement of the position coordinates of the elbow around the centerline over time, the arm swing is calculated based on the distance between the centerline and each displacement. When calculation is based on the relative position of multiple parts, such as stride length and arm swing, it is preferable to calculate based on the relationship between the most proximal parts (both ends of a single bone). In addition, the analysis unit 112 can calculate the back bending (forward leaning posture) based on a predetermined formula using, for example, the angle between the head and spine shoulder of the user 40 and the angle between the spine shoulder and spine base as indices. The angle between the spine shoulder and the spine base can be calculated based on a predetermined formula. The body's forward and backward wobble can be evaluated by evaluating the change over time of the angle of the forward leaning posture (trajectory of the angle transition). For the body sway angle, the analysis unit 112 can calculate the body sway angle based on the distance between the centerline and each displacement, using the angle between the spine base and the ground as an index, the X axis as a relative coordinate with the ground as the centerline, and the displacement of the position coordinates of the spine base around the centerline over time. Furthermore, the wobble of the body in the left-right direction can be evaluated by evaluating the change over time of the lateral sway angle (trajectory of the angle transition). Furthermore, the analysis unit 112 can evaluate the degree of interlocking of the waveform phases of each joint in the whole body, focusing on the point that the more combinations of the same phase, the higher the tendency that the user is developing a neuromuscular disease or the symptoms are progressing, and can calculate this based on a predetermined calculation formula. It can also be calculated based on a predetermined formula, focusing on the point that the fewer the combinations of inverse phase differences, the higher the tendency that the user has a neuromuscular disease or that the symptoms are progressing. Furthermore, the analysis unit 112 can calculate the phase difference between the upper and lower body, for example, based on the position coordinates of a predetermined part of the hand and a predetermined part of the foot. Here, the analysis unit 112 can also calculate numerical values referred to for evaluation related to multiple movements (e.g., walking and stair climbing) based on a single image acquired by the image acquisition unit 111. This can reduce the burden of exercise on the user, since the user need only perform a single movement for the evaluation of motor function, instead of multiple different movements.

Next, returning to FIG. 8, as step S103, the evaluation unit 113 evaluates the user's motor function. The details of the evaluation process are described below.

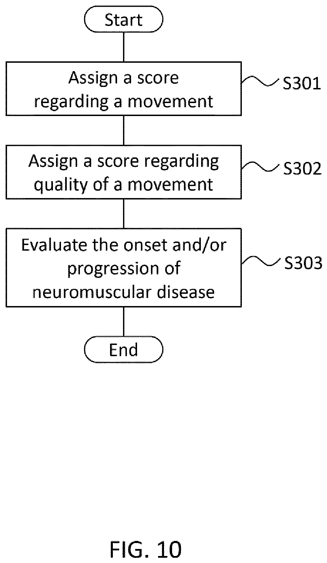

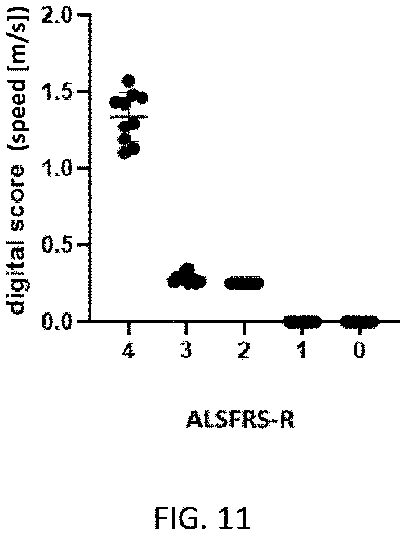

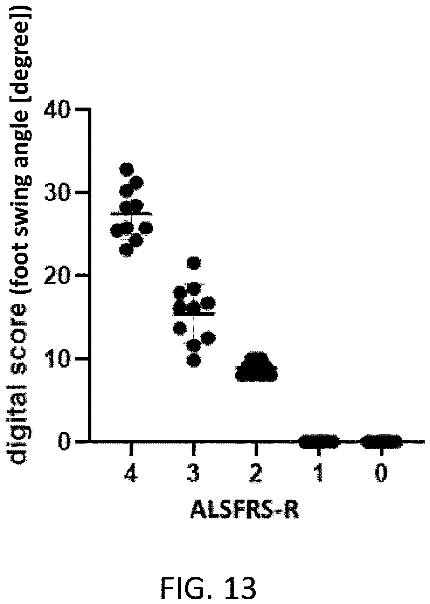

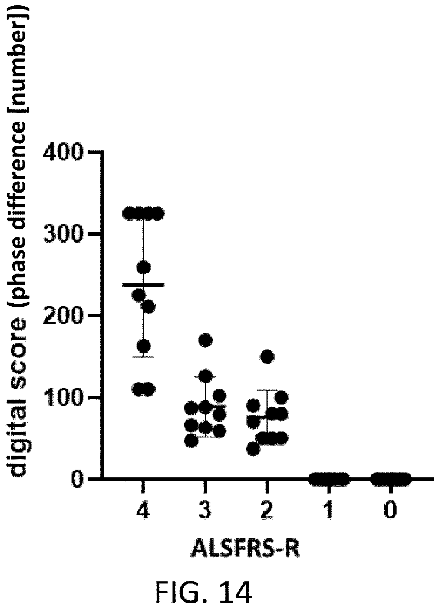

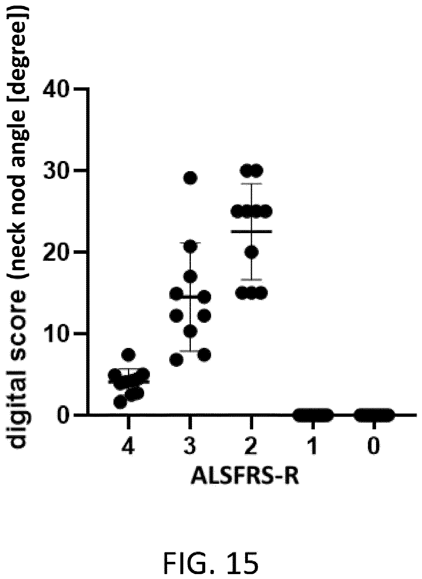

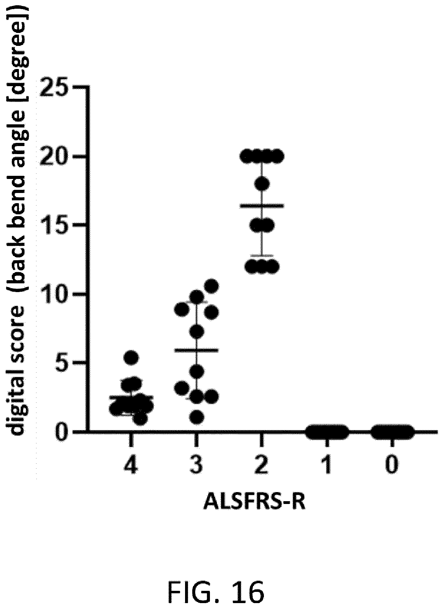

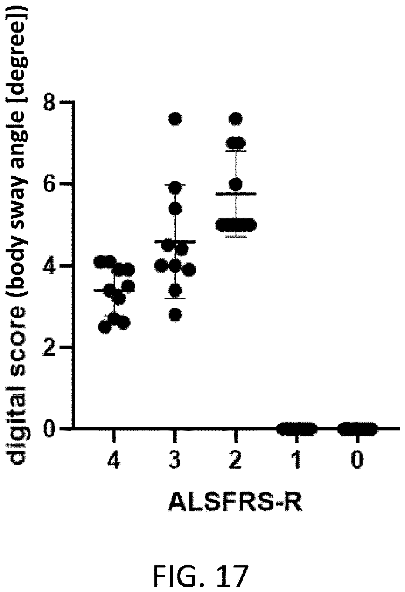

FIG. 10 shows a detailed example of the evaluation process by the analysis unit 112. As shown in step S301 in FIG. 10, as the evaluation process, first, the evaluation unit 113 refers to the numerical values calculated during the analysis process for evaluating significant differences in the movements of the user 40 and assigns scores for the significant differences in the movements. For example, if the movement is walking, the evaluation unit 113 evaluates the movement of walking as one of the items of the ALSFRS-R, an existing evaluation index used to evaluate ALS, as shown in FIG. 11. For the values calculated based on the formulas included in the above reference information for movement evaluation, based on the relationship between the numerical values calculated based on the calculation formula included in the above reference information for movement evaluation, a score such as 4 (normal walking), 3 (slight difficulty walking), 2 (assisted walking), 1 (unable to walk), or 0 (unable to move legs) is assigned as an evaluation of the ALSFRS-R items. Here, information determining the correspondence between the above values and scores can be stored as evaluation information in the evaluation information storage unit 134. Although the ALSFRS-R score is a score applied to users (ALS patients) after ALS diagnosis, in the examples shown in FIGs. 11 through 17, including this example, the users to be evaluated are users including healthy persons before ALS diagnosis, and for such users The ALSFRS-R score is applied. In this example, predicted values are applied for relatively low ALSFRS-R scores, such as 0, 1, and 2, and measured values are applied for relatively high ALSFRS-R scores, such as 3 and 4.

Next, as shown in step S302, the evaluation unit 113 refers to the numerical values calculated in the analysis unit to evaluate the quality of the user 40's movements, and scores the quality of the movements. For example, if the action is walking, the evaluation unit 113 assigns a score (e.g., for walking speed, an absolute score in the range of 0.0 m/s to 2.0 m/s) based on the numerical values of each of the digital evaluation items (if the action is walking, qualitative items such as walking speed, stride length, arm swing, back bend, body side swing, and phase difference between the upper and lower body), as shown in FIG. 11.

Next, as shown in step S303, the evaluation unit 113 evaluates whether or not the user 40 has developed and/or progressed to a neuromuscular disease based on the above assigned score. As a feature of this embodiment, the evaluation unit 113 can evaluate whether or not the disease has developed and/or the degree of progression of the disease based not only on the evaluation regarding the significant differences in the movements of the user 40, but also on the evaluation regarding the quality of the movements. For example, as shown in FIG. 11, in the case of ALS evaluation, for each given score for a movement associated with an item of the ALSFRS-R, each user's score for each of the quality of movement items, evaluated as a digital score, is plotted against the ALSFRS -R, it is possible to determine how the digital scores are distributed as an individual difference for each user within each score of the ALSFRS-R. The digital score for a particular user in a particular ALSFRS-R score can then be used as a reference to assess whether or not the user has ALS and/or the progression of ALS, and to determine which score is closest to another ALSFRS-R score within each of the user's ALSFRS-R scores. For example, in FIG. 11, a user with an ALSFRS-R score of "4" (normal walking) and a digital score (walking speed) of 1.5 m/s is unlikely to develop ALS, while a user with a digital score (walking speed) of 1.0 m/s can be judged to be close to "3" (somewhat difficult to walk) in ALSFRS-R score. Traditionally, the scores for movements associated with ALSFRS-R items are assigned a discrete score, such as "1" to "4," based on the physician's visual observation, whereas the digital score can use as a score the numerical values that represent movements such as walking speed, stride length, arm swing, back bend, lateral body swing, leg swing, neck nod, and phase difference of each joint in the body. As a result, even minor changes can be obtained as objective values. At the same time, by referring to the digital score in each ALSFRS-R score, the user can objectively predict the symptoms and progression of the disease between each ALSFRS-R score. In this step, the ALSFRS-R scores and digital scores can be output to the display 30, the output device 106, the user 40's terminal or the physician or other terminal. As shown in FIG. 11, information indicating the distribution of other users' digital scores on each ALSFRS-R score and the user 40's, digital score on the corresponding ALSFRS-R score can also be output.

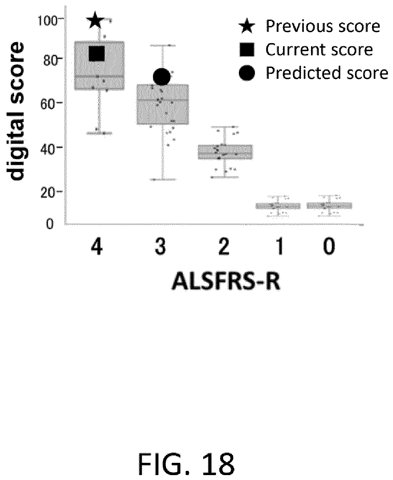

Based on the FIG. 18, which shows the relationship between the digital score and existing indices, the user's ALSFRS-R score can be predicted from the user's digital score. In FIG. 18, the results of the evaluation of the user's digital score can be displayed on the ALSFRS-R score, for example, the actual value of the user's previous digital score, the actual value of the current digital score, and the predicted value of the future digital score. By comparing the actual value of the user's digital score to a reference value (or range of reference digital scores) calculated based on the distribution of other users' digital scores, the ALSFRS-R score at a given point in time can be determined based on the actual value of the user's digital score, and also be predicted based on the previous actual value and the current actual value. In this way, by referring to the digital score together with the reference value, it is possible to predict the progression of symptoms and pathology of the disease more minimally than can be ascertained by the ALSFRS-R score. This device can also be used by non-specialists to understand the current status of symptoms of ALS patients in the same way as an ALS specialist. The above explanation is based on ALS as an example, but the symptoms and the speed of disease progression can be grasped for neuromuscular diseases other than ALS as well.

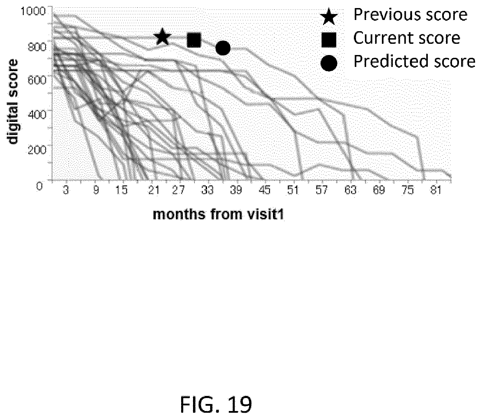

The digital score can also be displayed on a time axis, as shown in FIG. 19. For example, by displaying the monthly trends of the digital score, such as walking speed, and referring to its relationship to existing indexes such as the ALSFRS-R score, the user's ALSFRS-R score can be predicted based on the digital score, and the visually determine the degree of the user's disease progression based on the slope on the time axis. The larger the slope, the faster the disease progression, and the smaller the slope, the slower the progression can be ascertained. In this way, by showing the digital score on a time axis, the symptoms of the disease and the speed of the disease progression can be grasped. This device can also be used by non-specialists to understand the current status of symptoms of ALS patients in the same way as an ALS specialist. It can also be used to determine symptoms and the speed of disease progression for neuromuscular diseases other than ALS as well.

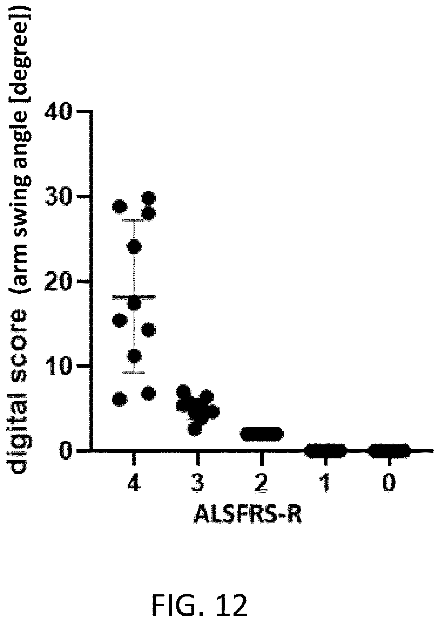

FIGS. 12 through 17 show the relationship between the ALSFRS-R scores and digital scores other than walking speed. For example, the angle of arm swing (arm swing), foot swing angle, phase difference (phase difference of each joint in the whole body), neck nod angle, back bend angle, and body sway angle can each be output. This can inform the user 40 which ALSFRS-R score the user is likely to shift to, based on the user's digital score. The evaluation unit 113 can also refer to the user's previous evaluation information stored in the evaluation information storage unit 134 and compare the previous evaluation with the current evaluation to evaluate the progression of ALS onset.

Furthermore, if treatment is being administered to the user, the effectiveness of the treatment can be checked based on the current and past evaluation information. The evaluation unit 113 can also identify the parts of the user's body that are affected by the motor dysfunction, particularly in relation to the evaluation of the quality of movement, and can stratify the patient with respect to motor dysfunction. In addition, the evaluation unit 113 can also evaluate muscle atrophy in connection with the quality of movement evaluation. Furthermore, the evaluation unit 113 can also perform an evaluation regarding motor function based on items related to cooperative movement (where "cooperative movement" refers to movement based on "nerve-muscle coordination" or "muscle-muscle coordination") of the parts of the user 40.

Neuromuscular diseases that can be evaluated by the device/system described herein include, for example, Parkinson's disease, Huntington's disease, amyotrophic lateral sclerosis, spinocerebellar degeneration, progressive supranuclear palsy, multiple system atrophy, multiple sclerosis, neuromyelitis optica, Adrenoleukodystrophy, metachromatic leukodystrophy, hyper glutaric acidemia type I, phenylketonuria, GM1 gangliosidosis, GM2 gangliosidosis, mucolipidosis type II (I-cell disease), Angelman syndrome, Krabbe disease, Batten disease, mucopolysaccharidosis, Rett disease, Niemann-Pick disease A,B,C, spinal cord injury, inclusion body myositis, myasthenia gravis, hereditary spastic paraplegia, primary lateral sclerosis, Charcot-Marie-Tooth disease, spinal muscular atrophy, Friedreich's ataxia, dermatomyositis, polymyositis, Guillain-Barre syndrome, chronic inflammatory, demyelinating polyneuritis, Lambert-Eaton myasthenia, multifocal motor neuropathy, anti-MAG peripheral neuropathy, facioscapulohumeral dystrophy, muscular dystrophy, myotonic dystrophy, Duchenne muscular dystrophy, facioscapulohumeral muscular dystrophy, spinal and bulbar atrophy, mitochondrial disease, Leigh's encephalomyelopathy, MELAS (Mitochondrial myopathy, Encephalopathy, Lactic acidosis, stroke-like episodes syndrome), fragile X-associated tremor/ataxia syndrome (FXTAS), Pelizaeus-Merzbacher disease (PMD), neuritis or myelitis caused by a virus or bacteria, cerebral infarction, cervical spondylosis, or spondylosis, etc. Although cerebral infarction, cervical spondylosis, and myelopathy are not generally included in neuromuscular diseases, they are diseases that cause movement disorders and shall be considered synonymously as diseases included in neuromuscular diseases for the purpose of this embodiment.

Furthermore, as a variation of this embodiment, a method of treating neuromuscular disease in a subject suspected of suffering from neuromuscular disease as a result of the above evaluation can be provided. In this example, in addition to the above described step of evaluating neuromuscular disease by imaging, a step of administering a neuromuscular disease therapeutic agent is included. The neuromuscular disease agents include, for example, drugs for ALS such as edaravone and riluzole, etc. drugs for spinocerebellar degeneration such as taltirelin and protirelin, etc. and drugs for Parkinson's disease such as L-dopa and apomorphine, etc.

Furthermore, as another variation of this embodiment, through the above evaluation, the drug efficacy in clinical trials can be evaluated (new clinical index/patient stratification). This can improve the success rate of clinical trials and realization of precision medicine. As yet another variation of this embodiment, diagnosis, consultation and medical treatment can be provided to the user based on the above evaluation. This enables a wide range of physicians to make early diagnosis of diseases such as ALS, as exemplified above for the user, and to realize early treatment intervention. As another variation of this embodiment, the above evaluation can be performed while administering medication and rehabilitation, thereby improving adherence to medication and the effectiveness of walking rehabilitation for the user. In other words, the drug effect can be detected with high sensitivity to the user, and the user's motivation for treatment and rehabilitation can be maintained and improved.

As described above, this embodiment enables the introduction of a highly objective, qualitative evaluation of movements as well as an evaluation based on existing movement items for the evaluation of predetermined movements to measure the onset and/or progression of neuromuscular diseases, etc. In addition, quantitative and highly accurate prediction of the onset and/or progression of neuromuscular diseases can be made by a non-invasive method, compared to the diagnosis previously made visually by physicians and others.

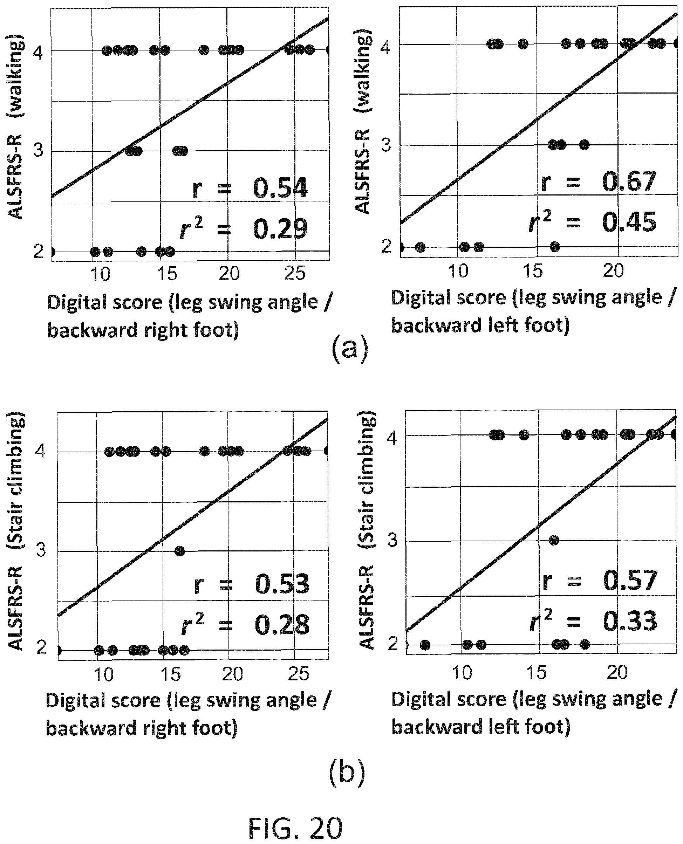

FIG. 20 shows an example of the results of the assessment of motor function (the relationship between the swing angle of the forward left right foot and the back left right foot and the ALSFRS-R score for walking).

As in this example, the forward and backward swing angles of the user's right foot and the forward and backward swing angles of the user's left foot can be used as reference information for evaluating the quality of movement in the evaluation process related to steps S301 through S303 above, which is performed by the evaluation unit 113.

As shown in FIG. 20(a), there is a relationship between the digital scores calculated based on the backward swing angle of the right foot and the backward swing angle of the left foot for each user (in this example, 15 ALS patients) and the ALSFRS-R scores for walking.

Similarly, for FIG. 20(b), the digital scores calculated based on the backward swing angle of the right foot and the backward swing angle of the left foot for each user (in this example, 15 ALS patients) and the ALSFRS-R scores for stair climbing are related. Although not shown in the figure, the correlation coefficient between the forward swing angle of the user's left foot and each ALSFRS-R score for walking and stair climbing tends to be lower than other qualitative evaluations (e.g., forward swing angle of the right foot and backward swing angle of the left and right feet).

This is thought to be an effect of the user's dominant foot. Thus, by measuring the backward swing angle of the user's right and left feet, a more objective qualitative evaluation of movement can be performed.

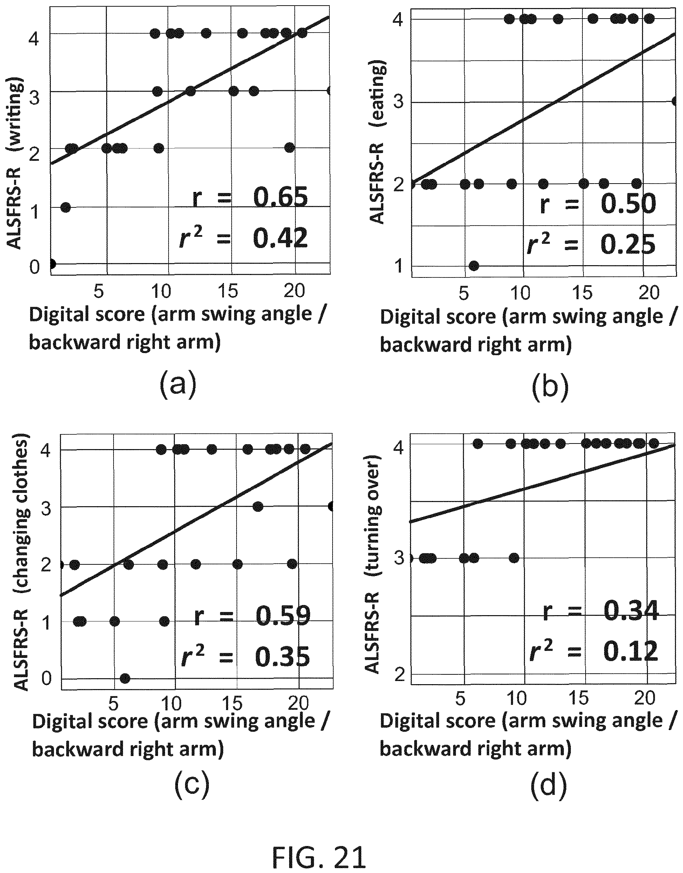

FIG. 21 shows an example of the results of the evaluation of motor function (the relationship between the swing angle of the upper limb and ALSFRS-R scores for writing, eating (e.g., feeding movements), changing clothes, and turning over in bed). As shown below, the establishment of digital scores for walking, stair climbing, as well as writing, eating, and changing clothes, and the ability to evaluate left-right and front-back movements for the upper limb as well as the lower limb, will allow evaluation of pathological progression that is less severe than evaluation using conventional ALSFRS-R scores.

FIG. 21(a) shows the relationship between the backward swing angle of the user's right hand and the ALSFRS-R score for writing, FIG. 21(b) shows the relationship between the backward swing angle of the user's right hand and the ALSFRS-R score for eating, and FIG. 21(c) shows the relationship between the backward swing angle of the user's right hand and the ALSFRS-R score for changing clothes, and FIG. 21(d) shows the relationship between the backward swing angle of the user's right hand and the ALSFRS-R score for turning over. As shown in each figure, it can be seen that there is a relationship between the user's upper extremity movements (especially the user's dominant arm) and the ALSFRS-R score. Thus, it is possible to evaluate the left/right and front/back movements of the user's upper limb. Here, the swing angle of the user's right hand mentioned above relates to the digital data obtained from the information obtained from the imaging of the user's walking motion. Thus, it is possible to evaluate the ALSFRS-R based on the digital data obtained from the user's walking motion, without the need to image different motions of the user's upper and lower limbs for each item of the ALSFRS-R.

Also not shown in the figure, as an example of the result of evaluation of a motor function, it is possible to find a correlation between the difference in the upward swing angle of the right hand and left hand in the backward direction and the ALSFRS-R score regarding walking, ascending and descending stairs.

Thus, digital evaluation of walking based on arm swing angles makes it possible to detect differences in left and right arm swing angles that cannot be evaluated visually, and the angle differences can be used as an index to evaluate, for example, ALS pathological progression in relation to walking and stair climbing.

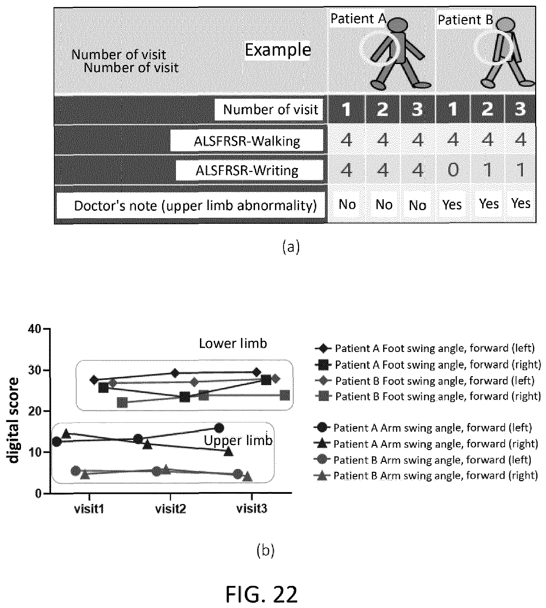

FIG. 22 illustrates the relationship between the user's upper extremity movements and the ALSFRS-R score for writing.

As shown in FIG. 22(a), the left and right arm swing angles analyzed from the three walking motions of user A and user B show no relationship between user A, who has no upper limb abnormality, and user B, who has an upper limb abnormality, in terms of the ALSFRS-R score for walking. However, there is a relationship between the ALSFRS-R scores for writing. Here, FIG. 22(b) shows the differences in digital scores based on the upper limbs (left and right arm swing angles) and lower limbs (left and right leg swing angles) of the same user A and user B. User B, who has an upper limb abnormality, shows no difference from normal user A in the digital score based on lower limb movements, but does show a difference in the digital score based on upper limb movements. Thus, it is possible to evaluate the ALSFRS-R score for writing based on the digital score based on the user's upper limb movements. As described above, it is possible to evaluate the ALSFRS-R score related to writing, etc. by focusing on the movements of the upper limbs among the digital data acquired by walking movements.

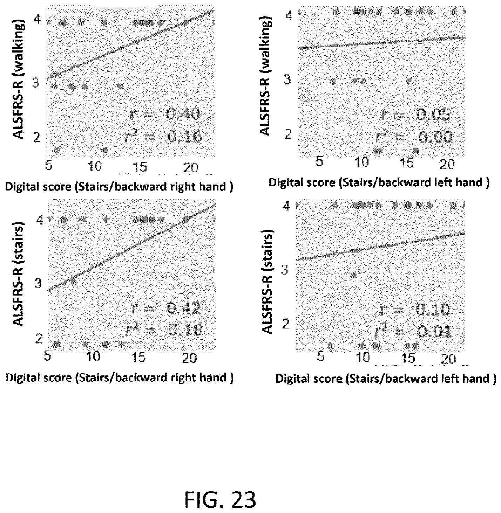

FIG. 23 shows the relationship between the digital score for stair climbing and the ALSFRS-R scores for walking and stair climbing.

As shown in FIG. 23, it is understood that the ALSFRS-R score for stair climbing based on the backward swing angle of the dominant right hand is related to the ALSFRS-R score for walking based on the same right hand backward swing angle. Similarly, although not shown, the ALSFRS-R score for stair climbing based on the backward swing angle of the dominant right foot is related to the ALSFRS-R score for walking based on the backward swing angle of the same right foot. Thus, digital evaluation of the backward swing angle of the dominant hand and foot can increase the accuracy of the evaluation and detect minor changes in pathological progression.

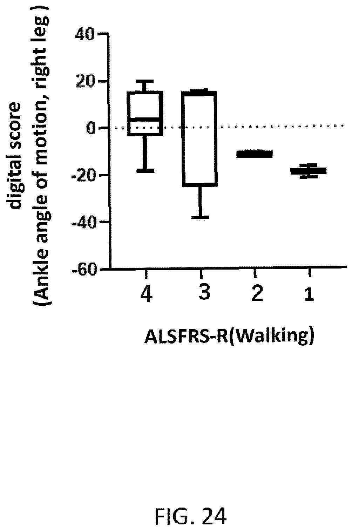

FIG. 24 shows an example of the results of the evaluation of motor function (the relationship between the angle of ankle motion and the ALSFRS-R score for walking).

In this example, the user's ankle movement angle can be used as the reference information for the quality of movement evaluation, especially in the user's walking movement, and as shown in FIG. 24, the digital score calculated based on the user's ankle movement angle and the ALSFRS-R score for walking. In addition, since the decrease in the user's ankle movement angle was detected prior to the decrease in the ALSFRS-R score for walking, measuring the user's ankle movement instead of the walking motion that requires the user to walk a certain distance is a simpler and more objective way to evaluate walking status and detect minor changes in pathological progression. In this example, as in the other examples, the digital evaluation improves the accuracy of the ALSFRS-R score compared to the conventional ALSFRS-R score based on visual observation, and enables the evaluation of minor pathological progression.

In the explanation using FIGs. 20 through 24, the description assumes the evaluation of ALS, but the evaluation of neuromuscular diseases based on the evaluation of the above-mentioned movements can also be targeted, and, for the evaluation of predetermined movements to measure the onset and/or progression of neuromuscular diseases, etc., a simpler, more objective, and more sensitive qualitative evaluation of movements can be introduced, in addition to the evaluation based on existing movement items.

### Description of reference numerals

1 Neuromuscular disease evaluation system
10 Server equipment
20 Camera
30 Display
40 User
NW Communication network

## Claims

1. A device for evaluating motor function related to a neuromuscular disease, comprising:
a memory unit for storing a reference value for evaluating a predetermined movement, which is calculated based on a position of at least one body part;
an analyzing unit for identifying a position of a body part of the user by analyzing information based on an image taken of the predetermined movement, including the user's body; and
an evaluation unit for evaluating the motor function based on a numerical value calculated based on the position of the body part of the user and the reference value.

2. The neuromuscular disease evaluation device according to claim 1, wherein the user's body part includes at least a part of the user's upper body.

3. The neuromuscular disease evaluation device according to claim 1, wherein the user's body part includes at least the parts of the user's upper and lower body.

4. The neuromuscular disease evaluation device as in any one of claims 2 or 3, wherein the upper body includes any one of the head, neck, back, and arms.

5. The neuromuscular disease evaluation device according to claim 1, wherein the evaluation unit evaluates the motor function based on claims related to the motor function in the examination of neuromuscular diseases.

6. The neuromuscular disease evaluation device according to claim 1, wherein the evaluation unit evaluates the motor function based on claims related to the motor functions in a test for amyotrophic lateral sclerosis.

7. The neuromuscular disease evaluation device according to claim 1, wherein the evaluation unit evaluates the motor function based on claims related to the coordinating movement of the user's parts.

8. The neuromuscular disease evaluation device according to claim 1, wherein the evaluation unit evaluates the motor function based on claims related to the motor function included in the ALS Functional Rating Scale (ALSFRS-R).

9. The neuromuscular disease evaluation device according to claim 1, wherein the evaluation unit includes evaluating for muscle atrophy.

10. The neuromuscular disease evaluation device according to claim 5, wherein the claim includes at least one of the following selected from language, salivation, swallowing, writing, eating actions, dressing activity/body movements, movements in bed, walking, climbing stairs, dyspnea, orthopnea, and respiratory failure.

11. The neuromuscular disease evaluation device according to claim 1,
wherein the analyzing unit analyzes an image of the user's body in the user's walking movement as the predetermined movement; and
the evaluation unit evaluates the walking movement and/or the quality of the walking movement as an evaluation of the motor function.

12. The neuromuscular disease evaluation device according to claim 11, wherein in connection with the evaluation of the quality of the walking movement, the user is evaluated for at least one of the following: walking speed, stride length, arm swing, leg swing, phase difference of each joint marker of the whole body, neck nod, back bend, body side swing, body wobble (front to back and side to side).

13. The neuromuscular disease evaluation device according to claim 11, wherein at least one of the following is evaluated in connection with the evaluation of the quality of the walking movement: walking speed, arm swing, leg swing, phase difference of each joint marker of the whole body, neck nodding, back bending, and body side swing.

14. The neuromuscular disease evaluation device according to claim 11, wherein the evaluation is performed in connection with the walking movements and with claims related to motor function included in the ALS Functional Rating Scale (ALSFRS-R).

15. The neuromuscular disease evaluation device according to claim 1, wherein the position relates to a joint.

16. The neuromuscular disease evaluation device according to claim 1,
wherein the analyzing unit analyzes an image of the user's body in the user's walking movement as the predetermined movement; and
the evaluation unit evaluates the writing, the eating, or the dressing/body movements and/or quality as the evaluation of the motor function.

17. The neuromuscular disease evaluation device according to claim 16, wherein the user's arm swing is evaluated in conjunction with the evaluation of the quality of writing, eating actions, or dressing activity/body movement activities.

18. The neuromuscular disease evaluation device according to claim 1, wherein the evaluation unit includes determining the degree of progression of the neuromuscular disease based on the evaluation.

19. The neuromuscular disease evaluation device according to claim 1, wherein the evaluation unit includes determining a patient group based on the progression of motor dysfunction based on the evaluation.

20. The neuromuscular disease evaluation device according to claim 1, wherein the neuromuscular disease includes amyotrophic lateral sclerosis.

21. The neuromuscular disease evaluation device according to claim 1, wherein the evaluation unit evaluates the range of motion of the joints.

22. The neuromuscular disease evaluation device according to claim 1, wherein the analysis unit includes identifying the body part of the user by motion capture technology.

23. The neuromuscular disease evaluation device according to claim 1, wherein the neuromuscular diseases include one of Parkinson's disease, Huntington's disease, amyotrophic lateral sclerosis, spinocerebellar degeneration, progressive supranuclear palsy, multiple system atrophy, multiple sclerosis, neuromyelitis optica, Adrenoleukodystrophy, metachromatic leukodystrophy, hyper glutaric acidemia type I, phenylketonuria, GM1 gangliosidosis, GM2 gangliosidosis, mucolipidosis type II (I-cell disease), Angelman syndrome, Krabbe disease, Batten disease, mucopolysaccharidosis, Rett disease, Niemann-Pick disease A,B,C, spinal cord injury, inclusion body myositis, myasthenia gravis, hereditary spastic paraplegia, primary lateral sclerosis, Charcot-Marie-Tooth disease, spinal muscular atrophy, Friedreich's ataxia, dermatomyositis, polymyositis, Guillain-Barre syndrome, chronic inflammatory, demyelinating polyneuritis, Lambert-Eaton myasthenia, multifocal motor neuropathy, anti-MAG peripheral neuropathy, facioscapulohumeral dystrophy, muscular dystrophy, myotonic dystrophy, Duchenne muscular dystrophy, facioscapulohumeral muscular dystrophy, spinal and bulbar atrophy, mitochondrial disease, Leigh's encephalomyelopathy, MELAS (Mitochondrial myopathy, Encephalopathy, Lactic acidosis, stroke-like episodes syndrome), fragile X-associated tremor/ataxia syndrome (FXTAS), Pelizaeus-Merzbacher disease (PMD), neuritis or myelitis caused by a virus or bacteria, cerebral infarction, cervical spondylosis, or spondylosis.

24. The neuromuscular disease evaluation device according to claim 16, wherein at least one of the following is evaluated in connection with the evaluation of the quality of the gait movement: the user's forward left-right foot and backward left-right foot swing, the forward left-right arm and backward right-right arm swing, and the angle of ankle motion.
